(19) Europäisches Patentamt
European Patent Office
Office européen des brevets

(11) Veröffentlichungsnummer: **0 179 018 A1**

(12) **EUROPÄISCHE PATENTANMELDUNG**

(21) Anmeldenummer: 85730143.6

(22) Anmeldetag: 16.10.85

(51) Int. Cl.[4]: C 07 C 43/29
C 07 C 43/174, C 07 C 149/273
C 07 D 317/54, C 07 C 93/14
C 07 D 213/64, A 01 N 31/14
A 01 N 33/08, A 01 N 43/40
A 01 N 43/30

(30) Priorität: 17.10.84 DE 3438483

(43) Veröffentlichungstag der Anmeldung: 23.04.86 Patentblatt 86/17

(84) Benannte Vertragsstaaten: AT BE CH DE FR GB IT LI LU NL SE

(71) Anmelder: SCHERING AKTIENGESELLSCHAFT Berlin und Bergkamen
Müllerstrasse 170/178 Postfach 65 03 11
D-1000 Berlin 65(DE)

(72) Erfinder: Franke, Helga, Dr.
Karl-Stieler-Strasse 2b
D-1000 Berlin 41(DE)

(72) Erfinder: Franke, Heinrich, Dr.
Fabeckstrasse 38
D-1000 Berlin 33(DE)

(72) Erfinder: Krüger, Hans-Rudolf, Dr.
Kulmbacherstrasse 15
D-1000 Berlin 30(DE)

(72) Erfinder: Joppien, Hartmut, Dr.
Juttastrasse 18
D-1000 Berlin 37(DE)

(54) **Substituierte Benzylether, Schädlingsbekämpfungsmittel enthaltend diese Verbindungen sowie Verfahren zu ihrer Herstellung.**

(57) Die Erfindung betrifft neue substituierte Benzylether der allgemeinen Formel

```
          CHF2
           |
R1 - C  - CH2 - X - CH - R4          (I),
     |                |
     R2               R3
```

in der

$R_1$ Aryl, durch $C_1$–$C_4$-Alkyl, Halogen-$C_1$–$C_4$-alkyl, Phenyl-$C_1$–$C_4$-alkyl, $C_2$–$C_4$-Alkenyl, Halogen-$C_2$–$C_4$-alkenyl, Phenyl-$C_2$–$C_4$-alkenyl, $C_2$–$C_4$-Alkinyl, Halogen-$C_2$–$C_4$-alkinyl, Phenyl-$C_2$–$C_4$-alkinyl, $C_1$–$C_4$-Alkoxy, Halogen-$C_1$–$C_4$-alkoxy, Phenyl-$C_1$–$C_4$-alkoxy, $C_2$–$C_4$-Alkenyloxy, Halogen-$C_2$–$C_4$-alkenyloxy, Phenyl-$C_2$–$C_4$-alkenyloxy, $C_2$–$C_4$-Alkinyloxy, Halogen-$C_2$–$C_4$-alkinyloxy, Phenyl-$C_2$–$C_4$-alkinyloxy, Alkylsulfonyloxy, Halogenalkylsulfonyloxy, Arylsulfonyloxy, Halogen, Cyan, Nitro, Aryloxy, Halogenaryloxy, $C_1$–$C_4$-Alkyl-aryloxy, Nitroaryloxy, substituiertes Aryl,

$R_2$ Wasserstoff oder $C_1$–$C_4$-Alkyl,

$R_3$ Wasserstoff, Cyano oder Ethinyl,

$R_4$ Phenyl, Phridyl oder durch $C_1$–$C_6$-Alkyl, Halogen-$C_1$–$C_6$-alkyl, Phenyl-$C_1$–$C_6$-alkyl, durch 0–, N– oder S-Atome unterbrochenes $C_1$–$C_6$-Alkyl, $C_2$–$C_4$-Alkenyl, Halogen-$C_2$–$C_4$-alkenyl, Phenyl-$C_2$–$C_4$-alkenyl, $C_1$–$C_4$-Alkoxy, Halogen-$C_1C_4$-alkoxy, Phenyl-$C_1$–$C_4$-alkoxy, $C_2$–$C_4$-Alkenyloxy, Halogen–$C_2$–$C_4$–Alkenyloxy, Phenyl-$C_2$–$C_4$-alkenyloxy, $C_2$–$C_4$-Alkinyloxy, Halogen-$C_2$–$C_4$-alkinyloxy, Phenyl–$C_2$–$C_4$-alkinyloxy, Aryloxy, Halogenaryloxy, $C_1$–$C_4$-Alkylaryloxy, Arylamino, Halogenarylamino, $C_1$–$C_4$-Alkylarylamino, Aryl-N-$C_1$–$C_4$-alkylamino, Aryl-N-$C_1$–$C_4$-acylamino, Aroyl, Halogenaroyl, $C_1$–$C_4$-Alkylaroyl, Aryl, Halogenaryl, $C_1$–$C_4$-Alkylaryl oder Halogen ein– oder mehrfach substituiertes Phenyl oder Pyridyl und

X Sauerstoff, Schwefel, Sulfinyl oder Sulfonyl darstellen, Schädlingsbekämpfungsmittel enthaltend diese Verbindungen sowie Verfahren zu ihrer Herstellung.

EP 0 179 018 A1

Die Erfindung betrifft neue substituierte Benzylether gemäß Oberbegriff von Anspruch 1, Verfahren gemäß Oberbegriff von Anspruch 49 zur Herstellung dieser Verbindungen sowie Schädlingsbekämpfungsmittel gemäß Oberbegriff von Anspruch 50 enthaltend diese Wirkstoffe.

Konstitutionsanaloge Wirkstoffe sind bereits bekannt (DE-OS 31 17 510).

Aufgabe der vorliegenden Erfindung ist die Schaffung neuer Wirkstoffe und eines diese Wirkstoffe enthaltenden Schädlingsbekämpfungsmittels, insbesondere eines Insektizides, mit überlegenen Eigenschaften.

Diese Aufgabe wird erfindungsgemäß durch die Bereitstellung von substituierten Benzylethern und eines Schädlingsbekämpfungsmittels, das mindestens eine Verbindung eines an solchen substituierten Benzylethers enthält, gelöst.

Diese substituierten Benzylether besitzen die allgemeine Formel

$$R_1 - \underset{\substack{| \\ R_2}}{\overset{\substack{CHF_2 \\ |}}{C}} - CH_2 - X - \underset{\substack{| \\ R_3}}{CH} - R_4 \qquad (I),$$

in der

$R_1$ Aryl, durch $C_1$-$C_4$-Alkyl, Halogen-$C_1$-$C_4$-alkyl, Phenyl-$C_1$-$C_4$-alkyl, $C_2$-$C_4$-Alkenyl, Halogen-$C_2$-$C_4$-alkenyl, Phenyl-$C_2$-$C_4$-alkenyl, $C_2$-$C_4$-Alkinyl, Halogen-$C_2$-$C_4$-alkinyl, Phenyl-$C_2$-$C_4$-alkinyl, $C_1$-$C_4$-Alkoxy, Halogen-$C_1$-$C_4$-alkoxy, Phenyl-$C_1$-$C_4$-alkoxy, $C_2$-$C_4$-Alkenyloxy, Halogen-$C_2$-$C_4$-alkenyloxy, Phenyl-$C_2$-$C_4$-alkenyloxy, $C_2$-$C_4$-Alkinyloxy,

Halogen-$C_2$-$C_4$-alkinyloxy, Phenyl-$C_2$-$C_4$-alkinyloxy, Alkylsulfonyloxy, Halogenalkylsulfonyloxy, Arylsulfonyloxy, Halogen, Cyan, Nitro, Aryloxy, Halogenaryloxy, $C_1$-$C_4$-Alkyl-aryloxy, Nitroaryloxy, substituiertes Aryl,

$R_2$ Wasserstoff oder $C_1$-$C_4$-Alkyl,

$R_3$ Wasserstoff, Cyano oder Ethinyl,

$R_4$ Phenyl, Pyridyl oder durch $C_1$-$C_6$-Alkyl, Halogen-$C_1$-$C_6$-alkyl, Phenyl-$C_1$-$C_6$-alkyl, durch O-, N- oder S-Atome unterbrochenes $C_1$-$C_6$-Alkyl, $C_2$-$C_4$-Alkenyl, Halogen-$C_2$-$C_4$-alkenyl, Phenyl-$C_2$-$C_4$-alkenyl, $C_1$-$C_4$-Alkoxy, Halogen-$C_1$-$C_4$-alkoxy, Phenyl-$C_1$-$C_4$-alkoxy, $C_2$-$C_4$-Alkenyloxy, Halogen-$C_2$-$C_4$-alkenyloxy, Phenyl-$C_2$-$C_4$-alkenyloxy, $C_2$-$C_4$-Alkinyloxy, Halogen-$C_2$-$C_4$-alkinyloxy, Phenyl-$C_2$-$C_4$-alkinyloxy, Aryloxy, Halogenaryloxy, $C_1$-$C_4$-Alkylaryloxy, Arylamino, Halogenarylamino, $C_1$-$C_4$-Alkylarylamino, Aryl-N-$C_1$-$C_4$-alkylamino, Aryl-N-$C_1$-$C_4$-acylamino, Aroyl, Halogenaroyl, $C_1$-$C_4$-Alkylaroyl, Aryl, Halogenaryl, $C_1$-$C_4$-Alkylaryl oder Halogen ein- oder mehrfach substituiertes Phenyl oder Pyridyl und

X Sauerstoff, Schwefel, Sulfinyl oder Sulfonyl darstellen.

Der in der allgemeinen Formel I als $R_1$ bezeichnete Arylrest umfaßt auch die Reste 1-Naphthyl, 2-Naphthyl, Benzofuran-5-yl, Benzothiophen-5-yl, Benzofuran-6-yl, Benzothiophen-6-yl, Benzoxazol-5-yl, Benzoxazol-6-yl, Indan-5-yl, Indan-6-yl, 1,4-Benzodioxan-6-yl, 1,3-Benzodioxan-6-yl, 1,3-Benzodioxan-7-yl und 1,3-Benzodioxol-5-yl.

Die erfindungsgemäßen Verbindungen haben insbesondere eine insektizide und akarizide Wirkung und sind somit zur Bekämpfung einer Vielfalt von Insekten und Milben einschließlich tierischer Ektoparasiten geeignet. Beispielsweise seien genannt

Lepidopteren wie Plutella xylostella, Spodoptera littoralis, Heliothis armigera und Pieris brassicae; Dipteren wie Musca domestica, Ceratitis capitata, Erioischia brassicae, Lucilia sericata und Aedes aegypti; Homopteren einschließlich Blattläusen wie Megoura viciae und Nilaparvata lugens; Coleopteren wie Phaedon cochleariae, Anthonomus grandis und Cornrootworm (Diabrotica spp., z.B. Diabrotica undecimpunctata); Orthopteren wie Blattella germanica; Zecken wie Boophilus microplus und Läuse wie Damalinia bovis und Linognathus vituli sowie Spinnmilben wie Tetranychus urticae und Panonychus ulmi.

Die Verbindungen zeichnen sich durch eine überraschend große Wirkung gegen wichtige Schädlingsarten aus, worin sie bekannten Schädlingsbekämpfungsmitteln gleicher Wirkungsrichtung überlegen sind.

Die Anwendung der erfindungsgemäßen Verbindungen kann in Konzentrationen von 0,0005 bis 5,0 %, vorzugsweise von 0,001 bis 0,1 % erfolgen, worunter das Gewicht in Gramm Wirkstoff in 100 ml Zubereitung zu verstehen ist.

Die erfindungsgemäßen Verbindungen können entweder allein, in Mischung miteinander oder mit anderen insektiziden Wirkstoffen angewendet werden. Gegebenenfalls können andere Pflanzenschutz- oder Schädlingsbekämpfungsmittel, wie zum Beispiel Insektizide, Akarizide oder Fungizide, je nach dem gewünschten Zweck, zugesetzt werden.

Eine Förderung der Wirkungsintensität und der Wirkungsgeschwindigkeit kann zum Beispiel durch wirkungssteigernde Zusätze, wie organische Lösungsmittel, Netzmittel und Öle erzielt werden. Solche Zusätze lassen daher gegebenenfalls eine Verringerung der Wirkstoffdosierung zu.

Als Mischungspartner können außerdem Phospholipide verwendet werden, zum Beispiel solche aus der Gruppe Phosphatidylcholin, den hydrierten Phosphatidylcholinen, Phosphatidylethanolamin, den N-Acyl-phosphatidylethanolaminen, Phosphatidylinosit, Phosphatidylserin, Lysolecithin und Phosphatidylglycerol.

Zweckmäßig werden die gekennzeichneten Wirkstoffe oder deren Mischungen in Form von Zubereitungen wie Pulvern, Streumitteln, Granulaten, Lösungen, Emulsionen oder Suspensionen, unter Zusatz von flüssigen und/oder festen Trägerstoffen, beziehungsweise Verdünnungsmitteln und gegebenenfalls Haft-, Netz-, Emulgier- und/oder Dispergierhilfsmitteln angewandt.

Geeignete flüssige Trägerstoffe sind zum Beispiel aliphatische und aromatische Kohlenwasserstoffe wie Benzol, Toluol, Xylol, Cyclohexanon, Isophoron, Dimethylsulfoxid, Dimethylformamid, weiterhin Mineralölfraktionen und Pflanzenöle.

Als feste Trägerstoffe eignen sich Mineralien, zum Beispiel Tonsil, Silicagel, Talkum, Kaolin, Attapulgit, Kalkstein, Kieselsäure und pflanzliche Produkte, zum Beispiel Mehle.

An oberflächenaktiven Stoffen sind zu nennen zum Beispiel Calciumligninsulfonat, Polyethylenalkylphenylether, Naphthalinsulfonsäuren und deren Salze, Phenolsulfonsäuren und

deren Salze, Formaldehydkondensate, Fettalkoholsulfate sowie substituierte Benzolsulfonsäuren und deren Salze.

Der Anteil des beziehungsweise der Wirkstoffe(s) in den verschiedenen Zubereitungen kann in weiten Grenzen variieren. Beispielsweise enthalten die Mittel etwa 10 bis 90 Gewichtsprozent Wirkstoffe, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen von etwa 100 bis 3000 Liter/ha. Eine Anwendung der Mittel im sogenannten Low-Volume und Ultra-Low-Volume-Verfahren ist ebenso möglich wie ihre Applikation in Form von sogenannten Mikrogranulaten.

Die Herstellung dieser Zubereitungen kann in an sich bekannter Art und Weise, zum Beispiel durch Mahl- oder Mischverfahren, durchgeführt werden. Gewünschtenfalls können die Einzelkomponenten auch erst kurz vor ihrer Verwendung gemischt werden, wie es zum Beispiel im sogenannten Tankmixverfahren in der Praxis durchgeführt wird.

Zur Herstellung der Zubereitungen werden zum Beispiel die folgenden Bestandteile eingesetzt:

A. 20 Gewichtsprozent Wirkstoff
35 Gewichtsprozent Bentonit
8 Gewichtsprozent Calciumsalz der Ligninsulfonsäure
2 Gewichtsprozent Natriumsalz des N-Methyl-N-oleyltaurins
35 Gewichtsprozent Kieselsäure

B. 20 Gewichtsprozent Wirkstoff
75 Gewichtsprozent Isophoron
5 Gewichtsprozent Kombinationsemulgator aus Calciumphenylsulfonat und Fettalkoholpolyglykolether

C. 80 Gewichtsprozent Wirkstoff
15 Gewichtsprozent Kaolin
5 Gewichtsprozent oberflächenaktive Stoffe auf Basis des Natriumsalzes des N-Methyl-N-oleyl-taurins und des Calciumsalzes der Ligninsulfonsäure

D. 45 Gewichtsprozent Wirkstoff
5 Gewichtsprozent Natriumaluminiumsilikat
15 Gewichtsprozent Cetylpolyglycolether mit 8 Mol Ethylenoxid
2 Gewichtsprozent Spindelöl
10 Gewichtsprozent Polyethylenglycol
23 Teile Wasser

Die erfindungsgemäßen Verbindungen lassen sich herstellen, indem man zum Beispiel

a) eine Verbindung der allgemeinen Formel

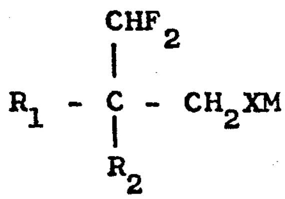

(II)

mit einer Verbindung der allgemeinen Formel

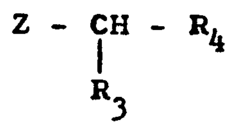

(III)

in Gegenwart einer Base und in Gegenwart eines Lösungsmittels umsetzt oder

b) eine Verbindung der allgemeinen Formel

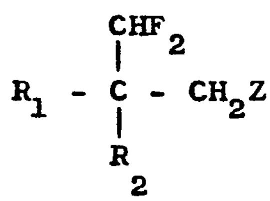

(IV)

mit einer Verbindung der allgemeinen Formel

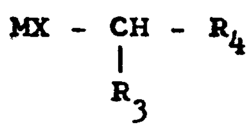

(V)

in Gegenwart einer Base und in Gegenwart eines Lösungsmittels umsetzt, worin $R_1$, $R_2$, $R_3$, $R_4$ und X die oben genannte Bedeutung haben, Z eine Fluchtgruppe wie zum Beispiel Halogen, Methansulfonat oder Toluolsulfonat und M Wasserstoff oder ein einwertiges Metalläquivalent darstellen.

Die Veretherung wird im allgemeinen in Lösung durchgeführt. Als Basen geeignet sind Metallalkoholate, wie zum Beispiel Kalium-tert.-butylat, Metallhydride, wie zum Beispiel Natriumhydrid, Metallamide, wie zum Beispiel Lithiumdiisopropylamid und Metallalkylverbindungen wie zum Beispiel Ethylmagnesiumbromid oder Butyllithium.

Als Lösungsmittel eignen sich gegenüber den Reaktanden, insbesondere den Basen, inerte Stoffe wie aliphatische und aromatische Kohlenwasserstoffe, wie zum Beispiel Hexan, Benzol oder Toluol und Ether, wie zum Beispiel Diethylether, Tetrahydrofuran oder Dimethoxyethan; geeignet sind ferner Amide wie Dimethylformamid, N-Methylpyrrolidon oder Hexamethylphosphorsäuretriamid.

Die Veretherung kann weiterhin in einem Zweiphasensystem unter Verwendung eines Katalysators und gegebenenfalls von Lösungsmitteln durchgeführt werden. Als Basen dienen Alkalihydroxide oder Alkalicarbonate, fest oder in wäßriger Lösung. Als Lösungsmittel geeignet sind die Reaktanden selbst, sofern sie flüssig sind; ansonsten werden mit Wasser nicht mischbare und gegenüber den Basen inerte Stoffe, wie aliphatische oder aromatische Kohlenwasserstoffe, wie zum Beispiel Hexan, Benzol oder Toluol verwendet. Als Katalysatoren kommen vorzugsweise Kronenether und quaternäre Ammoniumsalze in Frage.

Die Durchführung der Reaktionen erfolgt bei Temperaturen zwischen -78 °C und 140 °C, vorzugsweise bei 20 - 80 °C, in der Regel bei Normaldruck.

Die Verbindungen mit $R_3$ = Cyano werden aus den entsprechenden Ausgangsverbindungen hergestellt, bei denen $R_3$ Wasserstoff darstellt, welche mit N-Bromsuccinimid bromiert werden und man das Brom anschließend gegen Cyanid austauscht.

Die als Ausgangsstoffe zu verwendenden Alkohole lassen sich durch Reduktion der entsprechenden Nitrile, Aldehyde, Carbonsäuren beziehungsweise Carbonsäureester darstellen. Die Reduktion erfolgt nach an sich bekannten Methoden mit komplexen Metallhydriden, zum Beispiel Lithiumaluminiumhydrid oder Alkylaluminiumhydriden, zum Beispiel Diisobutylaluminiumhydrid. Die erforderlichen Nitrile lassen sich durch Alkylieren eines entsprechenden Arylacetonitrils mit $CHClF_2$ herstellen.

Die zu verwendenden Thioalkohole, Halogenide, Tosylate und Mesylate sind ebenfalls bereits bekannt oder können nach bereits bekannten Methoden hergestellt werden.

Die erfindungsgemäßen Verbindungen treten als optische Isomere auf. Die einzelnen Isomeren und ihre Mischungen gehören auch zum Gegenstand der Erfindung.

Das folgende Beispiel erläutert die Herstellung der erfindungsgemäßen Verbindungen.

Beispiel 1

2-(4-Chlorphenyl)-2-difluormethyl-1-(4-fluor-3-phenoxybenzyloxy)-propan

0,44 g (10 mmol) Natriumhydrid-Dispersion (55 %) werden durch dreimaliges Waschen mit trockenem Toluol vom Öl befreit. Es wird in 10 ml trockenem Dimethoxyethan suspendiert und dann werden unter Rühren nacheinander 1,77 g (8,0 mmol) 2-(4-Chlorphenyl)-2-difluormethyl-propanol und 2,24 g (8,0 mmol) 4-Fluor-3-phenoxybenzylbromid zugegeben. Nach fünfstündigem Rühren bei Raumtemperatur zeigt ein Dünnschichtchromatogramm die Abwesenheit der Ausgangsmaterialien an. Merck DC-Fertigplatten Kieselgel $60F_{254}$, Laufmittel Hexan/Essigester 1:1, $R_f$: 0,57.
Man gießt jetzt auf Eiswasser, extrahiert zweimal mit Ether, trocknet mit Magnesiumsulfat und dampft ein.
Nach Chromatographie mit Hexan/Essigester an Kieselgel verbleiben 2,96 g eines farblosen Öls, das sind 88 % der Theorie.

Brechungsindex $n_D^{20}$: 1,5570

| Analyse: | C | H | Cl | F |
|---|---|---|---|---|
| Berechnet: | 65,64 % | 4,80 % | 8,42 % | 13,54 % |
| Gefunden: | 65,67 % | 4,84 % | 8,66 % | 13,54 % |

In analoger Weise lassen sich die weiteren erfindungsgemäßen Verbindungen darstellen.

| Beispiel Nr. | Verbindung | Physikalische Konstante $n_D^{20}$ bzw. Fp. |
|---|---|---|
| 2 | 2-(4-Chlorphenyl)-2-difluormethyl-1-(3-phenoxy-benzyloxy)-propan | 1,5659 |
| 3 | 2-Difluormethyl-2-(4-methylphenyl)-1-(3-phenoxy-benzyloxy)-propan | 1,5596 |
| 4 | 2-Difluormethyl-1-(4-fluor-3-phenoxy-benzyloxy)-2-(4-methoxyphenyl-propan | 1,5581 |
| 5 | 2-Difluormethyl-2-(4-methoxyphenyl)-1-(3-phenoxy-benzyloxy)-propan | 1,5615 |
| 6 | 2-Difluormethyl-2-(4-fluorphenyl)-1-(4-fluor-3-phenoxy-benzyloxy)-propan | 1,5427 |
| 7 | 2-Difluormethyl-2-(4-fluorphenyl)-1-(3-phenoxy-benzyloxy)-propan | 1,5520 |
| 8 | 2-Difluormethyl-2-(4-ethoxyphenyl)-1-(3-phenoxy-benzyloxy-)-propan | 1,5575 |
| 9 | 2-Difluormethyl-2-(4-ethoxyphenyl)-1-(4-fluor-3-phenoxy-benzyloxy)-propan | 1,5486 |
| 10 | 2-Difluormethyl-2-(4-ethoxyphenyl)-1-(6-phenoxy-pyridin-2-ylmethoxy)-propan | 1,5557 |
| 11 | 2-Difluormethyl-2-(4-fluorphenyl)-1-(6-phenoxy-pyridin-2-ylmethoxy)-propan | 1,5502 |

0179018

| Beispiel Nr. | Verbindung | Physikalische Konstante $n_D^{20}$ bzw. Fp. |
|---|---|---|
| 12 | 2-Difluormethyl-1-(3-phenoxy-benzyloxy)-2-(4-n-propoxyphenyl)-propan | 1,5532 |
| 13 | 2-Difluormethyl-1-(4-fluor-3-phenoxy-benzyloxy)-2-(4-n-propoxyphenyl)-propan | 1,5439 |
| 14 | 2-Difluormethyl-2-(4-isopropoxyphenyl)-1-(3-phenoxy-benzyloxy)-propan | 1,5528 |
| 15 | 2-Difluormethyl-1-(4-fluor-3-phenoxy-benzyloxy)-2-(4-isopropoxyphenyl)-propan | 1,5440 |
| 16 | 2-(4-n-Butoxyphenyl)-2-difluormethyl-1-(3-phenoxy-benzyloxy)-propan | 1,5492 |
| 17 | 2-(4-n-Butoxyphenyl)-2-difluormethyl-1-(4-fluor-3-phenoxy-benzyloxy)-propan | 1,5410 |
| 18 | 2-(4-Allyloxyphenyl)-2-difluormethyl-1-(3-phenoxy-benzyloxy)-propan | 1,5615 |
| 19 | 2-(4-Allyloxyphenyl)-2-difluormethyl-1-(4-fluor-3-phenoxy-benzyloxy)-propan | 1,5530 |
| 20 | 2-Difluormethyl-2-[4-(2-fluorethoxy)-phenyl]-1-(3-phenoxybenzyloxy)-propan | 1,5550 |

| Beispiel Nr. | Verbindung | Physikalische Konstante $n_D^{20}$ bzw. Fp. |
|---|---|---|
| 21 | 2-Difluormethyl-1-(4-fluor-3-phenoxy-benzyloxy)-2-(4-propargyloxyphenyl)-propan | 1,5547 |
| 22 | 2-(4-Difluormethoxyphenyl)-2-difluormethyl-1-(3-phenoxy-benzyloxy)-propan | 1,5382 |
| 23 | 2-(4-Difluormethoxyphenyl)-2-difluormethyl-1-(4-fluor-3-phenoxy-benzyloxy)-propan | 1,5287 |
| 24 | 2-(4-Bromphenyl)-2-difluormethyl-1-(3-phenoxy-benzyloxy)-propan | 1,5767 |
| 25 | 2-(4-Bromphenyl)-2-difluormethyl-1-(4-fluor-3-phenoxy-benzyloxy)-propan | 1,5681 |
| 26 | 2-(4-tert.-Butylphenyl)-2-difluormethyl-1-(3-phenoxy-benzyloxy)-propan | 1,5484 |
| 27 | 2-(4-tert.-Butylphenyl)-2-difluormethyl-1-(4-fluor-3-phenoxy-benzyloxy)-propan | 1,5404 |
| 28 | 2-Difluormethyl-2-(2-naphthyl)-1-(3-phenoxy-benzyloxy)-propan | 1,5969 |
| 29 | 2-Difluormethyl-2-(2-naphthyl)-1-(4-fluor-3-phenoxy-benzyloxy)-propan | 1,5847 |
| 30 | 2-Difluormethyl-2-(3,4-dimethoxyphenyl)-1-(3-phenoxy-benzyloxy)-propan | 1,5602 |

| Beispiel Nr. | Verbindung | Physikalische Konstante $n_D^{20}$ bzw. Fp. |
|---|---|---|
| 31 | 2-Difluormethyl-2-(3,4-dimethoxyphenyl)-1-(4-fluor-3-phenoxy-benzyloxy)-propan | 1,5555 |
| 32 | 2-(1,3-Benzodioxol-5-yl)-2-difluormethyl-1-(3-phenoxy-benzyloxy)-propan | 1,5692 |
| 33 | 2-(1,3-Benzodioxol-5-yl)-2-difluormethyl-1-(4-fluor-3-phenoxy-benzyloxy)-propan | 1,5602 |
| 34 | 2-Difluormethyl-1-(4-fluor-3-phenoxy-benzyloxy)-2-[4-(2-fluorethoxy)-phenyl]-propan | 1,5460 |
| 35 | 2-Difluormethyl-1-[3-(2-fluorethoxy)-benzyloxy]-2-(4-ethoxyphenyl)-propan | 1,5240 |
| 36 | 2-Difluormethyl-1-[4-(2-fluorethoxy)-benzyloxy]-2-(4-ethoxyphenyl)-propan | 71-72 °C |
| 37 | 2-Difluormethyl-2-(4-ethoxyphenyl)-1-[3-(N-methylanilino)-benzyloxy]-propan | 1,5771 |
| 38 | 2-Difluormethyl-1-(3-phenoxy-benzyloxy)-2-[4-(2,2,2-trifluorethoxy)-phenyl]-propan | 1,5283 |
| 39 | 2-Difluormethyl-1-(4-fluor-3-phenoxy-benzyloxy)-2-[4-(2,2,2-trifluorethoxy)-phenyl]-propan | 1,5209 |

0179018

| Beispiel Nr. | Verbindung | Physikalische Konstante $n_D^{20}$ bzw. Fp. |
|---|---|---|
| 40 | 2-Difluormethyl-2-(4-ethoxyphenyl)-1-(3-phenoxy-benzylthio)-propan | 1,5783 |
| 41 | 2-Difluormethyl-2-(3,4-dimethoxyphenyl)-1-(6-phenoxypyridin-2-ylmethoxy)-propan | 81,5-83,5 °C |
| 42 | 2-(4-Bromphenyl)-2-difluormethyl-1-(6-phenoxy-pyridin-2-yl-methoxy)-propan | 1,5750 |
| 43 | 2-Difluormethyl-2-(2-naphthyl)-1-(6-phenoxypyridin-2-ylmethoxy)-propan | 1,5951 |
| 44 | 2-(4-Allyloxyphenyl)-2-difluormethyl-1-(6-phenoxy-pyridin-2-ylmethoxy)-propan | 1,5600 |
| 45 | 2-Difluormethyl-1-(6-phenoxy-pyridin-2-yl-methoxy)-2-(4-n-propoxyphenyl)-propan | 1,5509 |
| 46 | 2-Difluormethyl-2-(4-ethoxyphenyl)-1-(2-methyl-3-phenyl-benzyloxy)-propan | 1,5621 |

Die erfindungsgemäßen Verbindungen sind fast durchweg farb- und geruchlose Öle, die praktisch in allen organischen Lösungsmitteln gut löslich, in Wasser dagegen sehr schwer löslich sind.

Die folgenden Beispiele dienen zur Erläuterung der Anwendungsmöglichkeiten der erfindungsgemäßen Verbindungen, die in Form ihrer oben angeführten Zubereitungen erfolgte.

Beispiel 47

Abtötende Wirkung auf Junglarven der Kohlschabe (Plutella xylostella)

Die erfindungsgemäßen Verbindungen wurden als wäßrige Emulsionen mit der Wirkstoffkonzentration 0,1 % eingesetzt.

Mit diesen Wirkstoffzubereitungen wurden Blumenkohlblättchen in Polystyrol-Petrischalen dosiert (4 mg Spritzbrühe/$cm^2$) gespritzt. Nach dem Antrocknen der Spritzbeläge wurden in jede Petrischale 10 Jungraupen der Kohlschabe (Plutella xylostella) eingezählt und für 2 Tage in den geschlossenen Petrischalen dem behandelten Futter exponiert.

Kriterium für die Wirkungsbeurteilung war die Sterblichkeit der Raupen in % nach 2 Tagen. Die Ergebnisse sind in nachstehender Tabelle zusammengefaßt.

| Erfindungsgemäße Verbindungen | Wirkstoffkonzentration in % | Wirkung in % |
|---|---|---|
| 2-Difluormethyl-1-(3-phenoxy-benzyloxy)-2-(4-n-propoxyphenyl)-propan | 0,1 | 100 |
| 2-Difluormethyl-1-(4-fluor-3-phenoxy-benzyloxy)-2-(4-n-propoxyphenyl)-propan | 0,1 | 100 |
| 2-Difluormethyl-2-(4-isopropoxyphenyl)-1-(3-phenoxy-benzyloxy)-propan | 0,1 | 100 |
| 2-Difluormethyl-1-(4-fluor-3-phenoxy-benzyloxy)-2-(4-isopropoxyphenyl)-propan | 0,1 | 100 |
| 2-(4-n-Butoxyphenyl)-2-difluormethyl-1-(3-phenoxy-benzyloxy)-propan | 0,1 | 100 |

| Erfindungsgemäße Verbindungen | Wirkstoff-konzentration in % | Wirkung in % |
|---|---|---|
| 2-(4-Chlorphenyl)-2-difluormethyl-1-(4-fluor-3-phenoxy-benzyloxy)-propan | 0,1 | 100 |
| 2-(4-Chlorphenyl)-2-difluormethyl-1-(3-phenoxy-benzyloxy)-propan | 0,1 | 100 |
| 2-Difluormethyl-2-(4-methylphenyl)-1-(3-phenoxy-benzyloxy)-propan | 0,1 | 100 |
| 2-Difluormethyl-1-(4-fluor-3-phenoxy-benzyloxy)-2-(4-methoxyphenyl)-propan | 0,1 | 100 |
| 2-Difluormethyl-2-(4-methoxyphenyl)-1-(3-phenoxy-benzyloxy)-propan | 0,1 | 100 |
| 2-Difluormethyl-2-(4-fluorphenyl)-1-(4-fluor-3-phenoxy-benzyloxy)-propan | 0,1 | 100 |
| 2-Difluormethyl-2-(4-fluorphenyl)-1-(3-phenoxy-benzyloxy)-propan | 0,1 | 100 |
| 2-Difluormethyl-2-(4-ethoxyphenyl)-1-(3-phenoxy-benzyloxy)-propan | 0,1 | 100 |
| 2-Difluormethyl-2-(4-ethoxyphenyl)-1-(4-fluor-3-phenoxy-benzyloxy-propan | 0,1 | 100 |
| 2-Difluormethyl-2-(4-ethoxyphenyl)-1-(6-phenoxy-pyridin-2-ylmethoxy)-propan | 0,1 | 100 |

| Erfindungsgemäße Verbindungen | Wirkstoff-konzentration in % | Wirkung in % |
|---|---|---|
| 2-Difluormethyl-2-(4-fluorphenyl)-1-(6-phenoxy-pyridin-2-ylmethoxy)-propan | 0,1 | 100 |
| 2-Difluormethyl-2-(3,4-dimethoxyphenyl)-1-(3-phenoxy-benzyloxy)-propan | 0,1 | 65 |
| 2-Difluormethyl-2-(3,4-dimethoxyphenyl)-1-(4-fluor-3-phenoxy-benzyloxy)-propan | 0,1 | 94 |
| 2-(1,3-Benzodioxol-5-yl)-2-difluor-methyl-1-(3-phenoxybenzyloxy)-propan | 0,1 | 100 |
| 2-(1,3-Benzodioxol-5-yl)-2-difluormethyl-1-(4-fluor-3-phenoxy-benzyloxy)-propan | 0,1 | 65 |
| 2-Difluormethyl-2-(4-ethoxyphenyl)-1-[3-(N-methylanilino)-benzyloxy]-propan | 0,1 | 88 |
| 2-Difluormethyl-2-(2-naphthyl)-1-(3-phenoxy-benzyloxy)-propan | 0,1 | 100 |
| 2-Difluormethyl-2-(2-naphthyl)-1-(4-fluor-3-phenoxy-benzyloxy)-propan | 0,1 | 100 |

| Erfindungsgemäße Verbindungen | Wirkstoffkonzentration in % | Wirkung in % |
|---|---|---|
| 2-Difluormethyl-2-(2-naphthyl)-1-(6-phenoxy-pyridin-2-ylmethoxy)-propan | 0,1 | 90 |
| 2-(4-Difluormethoxyphenyl)-2-difluormethyl-1-(3-phenoxy-benzyloxy)-propan | 0,1 | 100 |
| 2-(4-Difluormethoxyphenyl)-2-difluormethyl-1-(4-fluor-3-phenoxy-benzyloxy)-propan | 0,1 | 100 |
| 2-(4-tert.-Butylphenyl)-2-difluormethyl-1-(3-phenoxy-benzyloxy)-propan | 0,1 | 60 |
| 2-(4-tert.-Butylphenyl)-2-difluormethyl-1-(4-fluor-3-phenoxy-benzyloxy)-propan | 0,1 | 70 |
| 2-Difluormethyl-2-(4-ethoxyphenyl)-1-(2-methyl-3-phenyl-benzyloxy)-propan | 0,1 | 85 |
| 2-Difluormethyl-1-(4-fluor-3-phenoxy-benzyloxy)-2-(4-propargyloxyphenyl)-propan | 0,1 | 100 |
| 2-Difluormethyl-1-(3-phenoxy-benzyloxy)-2-/4-(2,2,2-trifluorethoxy)-phenyl/-propan | 0,1 | 100 |
| 2-Difluormethyl-1-(4-fluor-3-phenoxy-benzyloxy)-2-/4-(2,2,2-trifluorethoxy)-phenyl/-propan | 0,1 | 100 |

| Erfindungsgemäße Verbindungen | Wirkstoff-konzentration in % | Wirkung in % |
|---|---|---|
| 2-Difluormethyl-2-[4-(2-fluorethoxy)-phenyl]-1-(3-phenoxybenzyloxy)-propan | 0,1 | 100 |
| 2-Difluormethyl-1-(4-fluor-3-phenoxybenzyloxy)-2-[4-(2-fluorethoxy)-phenyl]-propan | 0,1 | 100 |
| 2-Difluormethyl-1-[3-(2-fluorethoxy)-benzyloxy]-2-(4-ethoxyphenyl)-propan | 0,1 | 100 |
| 2-Difluormethyl-2-(4-ethoxyphenyl)-1-(3-phenoxy-benzylthio)-propan | 0,1 | 100 |
| 2-Difluormethyl-1-[4-(2-fluorethoxy)-benzyloxy]-2-(4-ethoxyphenyl)-propan | 0,1 | 100 |
| 2-(4-n-Butoxyphenyl)-2-difluormethyl-1-(4-fluor-3-phenoxy-benzyloxy)-propan | 0,1 | 100 |
| 2-(4-Allyloxyphenyl)-2-difluormethyl-1-(3-phenoxy-benzyloxy)-propan | 0,1 | 100 |
| 2-(4-Allyloxyphenyl)-2-difluormethyl-1-(4-fluor-3-phenoxy-benzyloxy)-propan | 0,1 | 100 |
| 2-Difluormethyl-2-(3,4-dimethoxyphenyl)-1-(6-phenoxypyridin-2-ylmethoxy)-propan | 0,1 | 65 |
| 2-(4-Bromphenyl)-2-difluormethyl-1-(6-phenoxy-pyridin-2-yl-methoxy)-propan | 0,1 | 100 |

| Erfindungsgemäße Verbindungen | Wirkstoff-konzentration in % | Wirkung in % |
|---|---|---|
| 2-(4-Bromphenyl)-2-difluormethyl-1-(3-phenoxy-benzyloxy)-propan | 0,1 | 100 |
| 2-(4-Bromphenyl)-2-dilfuormethyl-1-(4-fluor-3-phenoxy-benzyloxy)-propan | 0,1 | 100 |
| 2-(4-Allyloxyphenyl)-2-difluormethyl-1-(6-phenoxy-pyridin-2-ylmethoxy)-propan | 0,1 | 100 |
| 2-Difluormethyl-2-(4-ethoxyphenyl)-1-(2-methyl-3-phenyl-benzyloxy)-propan | 0,1 | 85 |

Beispiel 48

Abtötende Wirkung auf Larven (L2) der Ägyptischen Baumwolleule (Spodoptera littoralis)

Die erfindungsgemäßen Verbindungen sowie die Vergleichssubstanz wurden als wäßrige Emulsionen mit der Wirkstoffkonzentration 0,001 % eingesetzt. Mit diesen Wirkstoffzubereitungen wurden je ein Fiederblattpaar der Puffbohne (Vicia faba) sowie 10 Larven (L2) der Ägyptischen Baumwolleule (Spodoptera littoralis) pro Versuchsglied mit 4 mg Spritzbrühe/$cm^2$ in Polystyrol-Petrischalen dosiert gespritzt. Die geschlossenen Petrischalen wurden dann im Labor unter Langtagbedingungen für zwei Tage aufgestellt. Kriterium für die Wirkungsbeurteilung war die Mortalität der Larven in % nach zwei Tagen.

Die nachstehende Tabelle faßt die Ergebnisse zusammen.

| Erfindungsgemäße Verbindungen | Wirkstoffkonzentration in % | Wirkung in % |
|---|---|---|
| 2-Difluormethyl-2-(4-ethoxyphenyl)-1-(3-phenoxy-benzyloxy)-propan | 0,001 | 80 |
| 2-Difluormethyl-2-(4-ethoxyphenyl)-1-(4-fluor-3-phenoxy-benzyloxy)-propan | 0,001 | 100 |
| 2-(4-Difluormethoxyphenyl)-2-difluormethyl-1-(4-fluor-3-phenoxybenzyloxy)-propan | 0,001 | 100 |
| Vergleichsmittel gemäß DE-OS 31 17 510 | | |
| 2-(4-Ethoxyphenyl)-2-methyl-1-(3-phenoxy-benzyloxy)-propan | 0,001 | 55 |

Beispiel 49

Ovizide Wirkung auf Eigelege der Ägyptischen Baumwolleule (Spodoptera littoralis)

Die erfindungsgemäß zu verwendende Verbindung sowie die Vergleichssubstanz wurden als wäßrige Emulsionen mit den gewünschten Konzentrationen eingesetzt. In diese Wirkstoffzubereitung wurden 1 Tag alte Eigelege, die von befruchteten Falterweibchen auf Filterpapier abgesetzt worden waren, bis zur völligen Benetzung getaucht und für 4 Tage in geschlossenen Petrischalen deponiert. Kriterium für die Wirkungsbeurteilung war die prozentuale Schlupfverhinderung im Vergleich zu unbehandelten Eigelegen.
Die Ergebnisse sind in nachstehender Tabelle zusammengefaßt.

| Erfindungsgemäße Verbindung | Wirkstoffkonzentration in % | Wirkung in % |
|---|---|---|
| 2-Difluormethyl-2-(4-ethoxyphenyl)-1-(4-fluor-3-phenoxy-benzyloxy)-propan | 0,0025 | 90 |
| | 0,0010 | 50 |
| Vergleichsmittel gemäß DE-OS 31 17 510 | | |
| 2-(4-Ethoxyphenyl)-2-methyl-1-(3-phenoxy-benzyloxy)-propan | 0,0025 | 10 |
| | 0,0010 | 0 |

Beispiel 50

Abtötende Wirkung auf Junglarven der Kohlschabe (Plutella xylostella)

Die rfindungsgemäße Verbindung sowie die Vergleichssubstanz wurden als wäßrige Emulsionen mit den Wirkstoffkonzentrationen 0,0064; 0,0025; 0,001 eingesetzt. Mit diesen Wirkstoffzubereitungen wurden Blumenkohlblättchen in Polystyrol-Petrischalen dosiert (4 mg Spritzbrühe/$cm^2$) gespritzt. Nach dem Antrocknen der Spritzbeläge wurden in jede Petrischale 10 Jungraupen der Kohlschabe (Plutella xylostella) eingezählt und für 2 Tage in den geschlossenen Petrischalen dem behandelten Futter exponiert.

Kriterium für die Wirkungsbeurteilung war die Sterblichkeit der Raupen in % nach 2 Tagen. Die Ergebnisse sind in nachstehender Tabelle zusammengefaßt.

| Erfindungsgemäße Verbindung | Wirkstoff-konzentration in % | Wirkung in % |
|---|---|---|
| 2-Difluormethyl-2-(4-ethoxyphenyl)-1-(4-fluor-3-phenoxy-benzyloxy)-propan | 0,0064 | 100 |
| | 0,0025 | 100 |
| | 0,001 | 80 |
| 2-(4-Difluormethoxyphenyl)-2-difluormethyl-1-(4-fluor-3-phenoxy-benzyloxy)-propan | 0,0064 | 100 |
| | 0,0025 | 100 |
| | 0,001 | 80 |
| **Vergleichsmittel gemäß DE-OS 31 17 510** | | |
| 2-(4-Ethoxyphenyl)-2-methyl-1-(3-phenoxy-benzyloxy)-propan | 0,0064 | 90 |
| | 0,0025 | 70 |
| | 0,001 | 0 |

Beispiel 51

Abtötende Wirkung auf bewegliche Stadien und Eier der Grünen Bohnenspinnmilbe (Tetranychus urticae)

Die erfindungsgemäßen Verbindungen sowie die Vergleichssubstanz wurden als wäßrige Emulsionen mit der Wirkstoffkonzentration 0,1 % eingesetzt. Mit diesen Wirkstoffzubereitungen wurden getopfte und künstlich mit Spinnmilben (Tetranychus urticae) besetzte Buschbohnenpflanzen (Phaseolus vulgaris) im Primärblattstadium tropfnaß gespritzt und für 7 Tage im Labor aufgestellt. Danach wurde die Mortalität der beweglichen Stadien einerseits und der Eier andererseits mit Hilfe einer vergrößernden Lupe in Prozent geschätzt.

Die Ergebnisse sind in nachstehender Tabelle zusammengefaßt.

| Erfindungsgemäße Verbindungen | Wirkstoffkonzentration in % | Wirkung in % auf bewegliche Stadien Eier von Tetranychus urticae |
|---|---|---|
| 2-(4-Chlorphenyl)-2-difluormethyl-1-(4-fluor-3-phenoxy-benzyloxy)-propan | 0,1 | 95 / 40 |
| 2-Difluormethyl-2-(4-fluorphenyl)-1-(4-fluor-3-phenoxy-benzyloxy)-propan | 0,1 | 100 / 0 |
| 2-Difluormethyl-2-(4-ethoxyphenyl)-1-(4-fluor-3-phenoxy-benzyloxy)-propan | 0,1 | 75 / 25 |
| 2-Difluormethyl-2-(4-fluorphenyl)-1-(6-phenoxy-pyridin-2-ylmethoxy)-propan | 0,1 | 93 / 94 |
| 2-(4-Difluormethoxyphenyl)-2-difluormethyl-1-(4-fluor-3-phenoxybenzyloxy)-propan | 0,1 | 100 / 0 |
| Vergleichsmittel gemäß DE-OS 31 17 510 | | |
| 2-(4-Ethoxyphenyl)-2-methyl-1-(3-phenoxy-benzyloxy)-propan | 0,1 | 38 4 |

Beispiel 52

Insektizide bzw. akarizide Wirkung gegen Boophilus microplus (1), Lucilia sericata (2), Musca domestica (3) und Blatella germanica (4)

(1) Filterpapier (9 cm Ø) wird mit 1 ml eines aliquoten Teiles einer Lösung der Testsubstanz in Aceton bei verschiedenen Konzentrationen getränkt. Nach Trocknung werden die Filterpapiere zu Umschlägen gefaltet, in welche Zecken-Larven (Boophilus microplus) eingebracht und für 48 Stunden bei 25 °C und 80 % Raumfeuchte gehalten werden. Die prozentuale Sterberate der Schädlingslarven wird dann festgestellt und mit den Kontrollversuchen verglichen.

Die Kontrollversuche ergaben eine Mortalität von $<5$ %, währenddessen die Verbindungen gemäß den Beispielen 4, 5, 8, 10, 11, 17, 24, 25, 27, 32, 42 und 45 eine mindestens 50 %ige Mortalität bei einer Konzentration von 100 ppm ergaben.

(2) 1 ml eines aliquoten Teiles einer Lösung der Testverbindung in Aceton bei verschiedenen Konzentrationen wird auf Baumwoll-Dentalrollen (1 cm x 2 cm) aufgebracht, die sich in Glasröhrchen (2 cm Ø und 5 cm Länge) befinden. Nach Trocknung wird das so behandelte Material mit 1 ml einer Nährlösung getränkt, die von Junglarven der Schafschmeißfliege (Lucilia sericata) durchsetzt ist. Dann werden die Glasröhrchen mit einem Baumwollstopfen verschlossen und für 24 Stunden bei 25 °C gehalten. Bei Kontrollversuchen war die Mortalität $<5$ %, währenddessen die Verbindungen gemäß den Beispielen 4, 5, 8, 9, 10, 32 und 33 eine $LC_{50}$ bei weniger als 100 ppm aufwiesen.

(3) Aliquote Teile einer Lösung der Testsubstanz in Aceton in verschiedenen Konzentrationen werden auf Filterpapier (9 cm Ø) in Petrischalen (9 cm Ø) mit Uhrglasabdeckung aufgebracht. Nach Verdampfen des Lösungsmittels werden die so behandelten Oberflächen zusammen mit Kontrollversuchen mit Aceton allein ausgewachsenen Hausfliegen (Musca domestica) exponiert und für 24 Stunden bei 22 °C gehalten.

Die prozentuale Mortalität wird anschließend durch Auszählen bestimmt. Bei Kontrollversuchen war die Mortalität <5 %, währenddessen die Verbindungen gemäß den Beispielen 4, 9, 15, 25, 38 und 39 eine $LD_{50}$ bei weniger als 100 mg/$m^2$ aufwiesen.

(4) Aliquote Teile einer Lösung der Testsubstanz in Aceton in verschiedenen Konzentrationen werden auf Glasplatten (10 x 10 cm) aufgebracht. Nach Verdampfen des Lösungsmittels werden die so behandelten Oberflächen zusammen mit Kontrollversuchen mit Aceton allein mit Junglarven (L 2) der Deutschen Schabe (Blattella germanica) exponiert, indem sie durch mit Poly-Tetrafluorethylen bedampfte Glasringe (6 cm Ø) auf der behandelten Oberfläche für 24 Stunden bei 22 °C gehalten werden. Die prozentuale Mortalität der Insekten wird anschließend durch Auszählen bestimmt.

Bei den Kontrollversuchen war die Mortalität 5 %, währenddessen die Verbindungen gemäß den Beispielen 4, 5, 8, 9, 10, 11, 17, 18, 19, 22, 23, 24, 32, 33, 38 und 39 eine $LD_{50}$ bei weniger als 100 mg/$m^2$ zeigten.

## Patentansprüche

1. Substituierte Benzylether der allgemeinen Formel

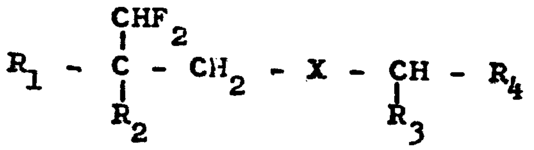

(I),

in der

$R_1$ Aryl, durch $C_1$-$C_4$-Alkyl, Halogen-$C_1$-$C_4$-alkyl, Phenyl-$C_1$-$C_4$-alkyl, $C_2$-$C_4$-Alkenyl, Halogen-$C_2$-$C_4$-alkenyl, Phenyl-$C_2$-$C_4$-alkenyl, $C_2$-$C_4$-Alkinyl, Halogen-$C_2$-$C_4$-alkinyl, Phenyl-$C_2$-$C_4$-alkinyl, $C_1$-$C_4$-Alkoxy, Halogen-$C_1$-$C_4$-alkoxy, Phenyl-$C_1$-$C_4$-alkoxy, $C_2$-$C_4$-Alkenyloxy, Halogen-$C_2$-$C_4$-alkenyloxy, Phenyl-$C_2$-$C_4$-alkenyloxy, $C_2$-$C_4$-Alkinyloxy, Halogen-$C_2$-$C_4$-alkinyloxy, Phenyl-$C_2$-$C_4$-alkinyloxy, Alkylsulfonyloxy, Halogenalkylsulfonyloxy, Arylsulfonyloxy, Halogen, Cyan, Nitro, Aryloxy, Halogenaryloxy, $C_1$-$C_4$-Alkyl-aryloxy, Nitroaryloxy, substituiertes Aryl,

$R_2$ Wasserstoff oder $C_1$-$C_4$-Alkyl,

$R_3$ Wasserstoff, Cyano oder Ethinyl,

$R_4$ Phenyl, Pyridyl oder durch $C_1$-$C_6$-Alkyl, Halogen-$C_1$-$C_6$-alkyl, Phenyl-$C_1$-$C_6$-alkyl, durch O-, N- oder S-Atome unterbrochenes $C_1$-$C_6$-Alkyl, $C_2$-$C_4$-Alkenyl, Halogen-$C_2$-$C_4$-alkenyl, Phenyl-$C_2$-$C_4$-alkenyl, $C_1$-$C_4$-Alkoxy, Halogen-$C_1$-$C_4$-alkoxy, Phenyl-$C_1$-$C_4$-alkoxy, $C_2$-$C_4$-Alkenyloxy, Halogen-$C_2$-$C_4$-alkenyloxy, Phenyl-$C_2$-$C_4$-alkenyloxy, $C_2$-$C_4$-Alkinyloxy, Halogen-$C_2$-$C_4$-alkinyloxy, Phenyl-$C_2$-$C_4$-alkinyloxy, Aryloxy, Halogenaryloxy, $C_1$-$C_4$-Alkylaryloxy, Arylamino, Halogenarylamino, $C_1$-$C_4$-Alkylarylamino, Aryl-N-$C_1$-$C_4$-alkylamino, Aryl-N-$C_1$-$C_4$-acylamino, Aroyl, Halogenaroyl, $C_1$-$C_4$-Alkylaroyl, Aryl, Halogenaryl, $C_1$-$C_4$-Alkylaryl oder Halogen ein- oder mehrfach substituiertes Phenyl oder Pyridyl und

X Sauerstoff, Schwefel, Sulfinyl oder Sulfonyl darstellen.

2. Substituierte Benzylether gemäß Anspruch 1, worin

$R_1$ Chlorphenyl, Bromphenyl, Fluorphenyl, Methylphenyl, Methoxyphenyl, Ethoxyphenyl, Difluormethoxyphenyl, 1,3-Benzodioxol-5-yl oder Dimethoxyphenyl,

$R_2$ Methyl,

$R_3$ Wasserstoff,

$R_4$ Phenoxyphenyl, Fluor-phenoxyphenyl oder Phenoxypyridyl und

X Sauerstoff darstellen.

3. 2-(4-Chlorphenyl)-2-difluormethyl-1-(4-fluor-3-phenoxy-benzyloxy)-propan.

4. 2-(4-Chlorphenyl)-2-difluormethyl-1-(3-phenoxy-benzyloxy)-propan.

5. 2-Difluormethyl-2-(4-methylphenyl)-1-(3-phenoxy-benzyloxy)-propan.

6. 2-Difluormethyl-1-(4-fluor-3-phenoxybenzyloxy)-2-(4-methoxyphenyl)-propan.

7. 2-Difluormethyl-2-(4-methoxyphenyl)-1-(3-phenoxy-benzyloxy)-propan.

8. 2-Difluormethyl-2-(4-fluorphenyl)-1-(4-fluor-3-phenoxy-benzyloxy)-propan.

9. 2-Difluormethyl-2-(4-fluorphenyl)-1-(3-phenoxy-benzyloxy)-propan.

10. 2-Difluormethyl-2-(4-ethoxyphenyl)-1-(3-phenoxy-benzyloxy)-propan.

11. 2-Difluormethyl-2-(4-ethoxyphenyl)-1-(4-fluor-3-phenoxy-benzyloxy)-propan.

12. 2-Difluormethyl-2-(4-ethoxyphenyl)-1-(6-phenoxy-pyridin-2-ylmethoxy)-propan.

13. 2-Difluormethyl-2-(4-fluorphenyl)-1-(6-phenoxy-pyridin-2-ylmethoxy)-propan.

14. 2-Difluormethyl-1-(3-phenoxy-benzyloxy)-2-(4-n-propoxyphenyl)-propan.

15. 2-Difluormethyl-1-(4-fluor-3-phenoxybenzyloxy)-2-(4-n-propoxyphenyl)-propan.

16. 2-Difluormethyl-2-(4-isopropoxyphenyl)-1-(3-phenoxy-benzyloxy)-propan.

17. 2-Difluormethyl-1-(4-fluor-3-phenoxy-benzyloxy)-2-(4-isopropoxyphenyl)-propan.

18. 2-(4-n-Butoxyphenyl)-2-difluormethyl-1-(3-phenoxy-benzyloxy)-propan.

19. 2-(4-n-Butoxyphenyl)-2-difluormethyl-1-(4-fluor-3-phenoxy-benzyloxy)-propan.

20. 2-(4-Allyloxyphenyl)-2-difluormethyl-1-(3-phenoxy-benzyloxy)-propan.

21. 2-(4-Allyloxyphenyl)-2-difluormethyl-1-(4-fluor-3-phenoxy-benzyloxy)-propan.

22. 2-Difluormethyl-2-/4-(2-fluorethoxy)-phenyl7-1-(3-phenoxybenzyloxy)-propan

23. 2-Difluormethyl-1-(4-fluor-3-phenoxy-benzyloxy)-2-(4-propargyloxyphenyl)-propan.

24. 2-(4-Difluormethoxyphenyl)-2-difluormethyl-1-(3-phenoxy-benzyloxy)-propan.

25. 2-(4-Difluormethoxyphenyl)-2-difluormethyl-1-(4-fluor-3-phenoxy-benzyloxy)-propan.

26. 2-(4-Bromphenyl)-2-difluormethyl-1-(3-phenoxy-benzyloxy)-propan.

27. 2-(4-Bromphenyl)-2-difluormethyl-1-(4-fluor-3-phenoxy-benzyloxy)-propan.

28. 2-(4-tert.-Butylphenyl)-2-difluormethyl-1-(3-phenoxy-benzyloxy)-propan.

29. 2-(4-tert.-Butylphenyl)-2-difluormethyl-1-(4-fluor-3-phenoxy-benzyloxy)-propan.

30. 2-Difluormethyl-2-(2-naphthyl)-1-(3-phenoxy-benzyloxy)-propan.

31. 2-Difluormethyl-2-(2-naphthyl)-1-(4-fluor-3-phenoxy-benzyloxy)-propan.

32. 2-Difluormethyl-2-(3,4-dimethoxyphenyl)-1-(3-phenoxy-benzyloxy)-propan.

33. 2-Difluormethyl-2-(3,4-dimethoxyphenyl)-1-(4-fluor-3-phenoxy-benzyloxy)-propan.

34. 2-(1,3-Benzodioxol-5-yl)-2-difluormethyl-1-(3-phenoxy-benzyloxy)-propan.

35. 2-(1,3-Benzodioxol-5-yl)-2-difluormethyl-1-(4-fluor-3-phenoxy-benzyloxy)-propan.

36. 2-Difluormethyl-1-(4-fluor-3-phenoxybenzyloxy)-2-/4-(2-fluorethoxy)-phenyl/-propan.

37. 2-Difluormethyl-1-/3-(2-fluorethoxy)-benzyloxy/-2-(4-ethoxyphenyl)-propan.

38. 2-Difluormethyl-1-/4-(2-fluorethoxy)-benzyloxy/-2-(4-ethoxyphenyl)-propan.

39. 2-Difluormethyl-2-(4-ethoxyphenyl)-1-/3-(N-methyl-anilino)-benzyloxy/-propan.

40. 2-Difluormethyl-1-(3-phenoxy-benzyloxy)-2-/4-(2,2,2-trifluorethoxy)-phenyl/-propan.

41. 2-Difluormethyl-1-(4-fluor-3-phenoxy-benzyloxy)-2-/4-(2,2,2-trifluorethoxy)-phenyl/-propan.

42. 2-Difluormethyl-2-(4-ethoxyphenyl)-1-(3-phenoxy-benzylthio)-propan.

43. 2-Difluormethyl-2-(3,4-dimethoxyphenyl)-1-(6-phenoxy-pyridin-2-ylmethoxy)-propan.

44. 2-(4-Bromphenyl)-2-difluormethyl-1-(6-phenoxy-pyridin-2-yl-methoxy)-propan.

45. 2-Difluormethyl-2-(2-naphthyl)-1-(6-phenoxy-pyridin -2-ylmethoxy)-propan.

46. 2-(4-Allyloxyphenyl)-2-difluormethyl-1-(6-phenoxy-pyridin-2-ylmethoxy)-propan.

47. 2-Difluormethyl-1-(6-phenoxy-pyridin-2-ylmethoxy)-2-(4-n-propoxyphenyl)-propan.

48. 2-Difluormethyl-2-(4-ethoxyphenyl)-1-(2-methyl-3-phenyl-benzyloxy)-propan.

49. Verfahren zur Herstellung von substituierten Benzylethern gemäß Ansprüchen 1 bis 48, dadurch gekennzeichnet, daß man

a) eine Verbindung der allgemeinen Formel

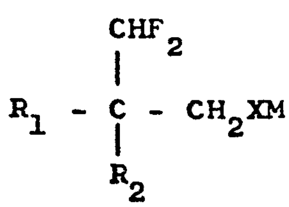

(II)

mit einer Verbindung der allgemeinen Formel

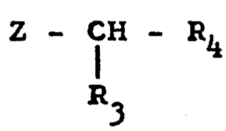

(III)

in Gegenwart einer Base und in Gegenwart eines Lösungsmittels umsetzt oder

b) eine Verbindung der allgemeinen Formel

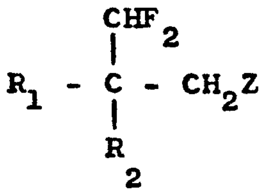

(IV)

mit einer Verbindung der allgemeinen Formel

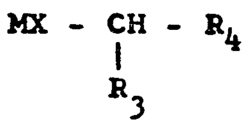

(V)

in Gegenwart einer Base und in Gegenwart eines Lösungsmittels umsetzt, worin $R_1$, $R_2$, $R_3$, $R_4$ und X die oben genannte Bedeutung haben, Z eine Fluchtgruppe wie zum Beispiel Halogen, Methansulfonat oder Toluolsulfonat und M Wasserstoff oder ein einwertiges Metalläquivalent darstellen.

50. Schädlingsbekämpfungsmittel, gekennzeichnet durch einen Gehalt an mindestens einer Verbindung gemäß Ansprüchen 1 bis 48.

51. Schädlingsbekämpfungsmittel gemäß Anspruch 50 zur Bekämpfung von Insekten.

52. Schädlingsbekämpfungsmittel gemäß Anspruch 50 in Mischung mit Träger- und/oder Hilfsstoffen.

Europäisches Patentamt

# EUROPÄISCHER RECHERCHENBERICHT

0179018

Nummer der Anmeldung

EP 85730143.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| D,A | DE - A1 - 3 117 510 (MITSUI TOATSU)<br>* Ansprüche 1,8,12 *<br>-- | 1,49,50 | C 07 C 43/29<br>C 07 C 43/174<br>C 07 C 149/273<br>C 07 D 317/54<br>C 07 C 93/14<br>C 07 D 213/64<br>A 01 N 31/14<br>A 01 N 33/06<br>A 01 N 43/40<br>A 01 N 43/30 |
| A | DE - A1 - 3 402 483 (MITSUI TOATSU)<br>* Ansprüche 1,6,10 *<br>-- | 1,49,50 | |
| A | DE - A1 - 3 337 673 (MITSUI TOATSU)<br>* Anspruch 1 *<br>-- | 1,49 | |
| A | DE - A1 - 3 139 976 (MITSUI TOATSU)<br>* Ansprüche 1,9,13 *<br>-- | 1,49,50 | |
| A | EP - A1 - 0 094 085 (SUMITOMO CHEMICAL)<br>* Ansprüche 1,14,17 *<br>-- | 1,49,50 | RECHERCHIERTE SACHGEBIETE (Int. Cl. 4) |
| A | DD - A - 157 254 (ELI LILLY)<br>* Seite 1; Anspruch 1 *<br>---- | 1,50 | C 07 C 43/00<br>C 07 C 149/00<br>C 07 D 317/00<br>C 07 D 213/00<br>C 07 C 93/00 |

Der vorliegende Recherchenbericht wurde für alle Patentansprüche erstellt

| Recherchenort | Abschlußdatum der Recherche | Prüfer |
|---|---|---|
| WIEN | 10-01-1986 | REIF |

KATEGORIE DER GENANNTEN DOKUMENTEN

X von besonderer Bedeutung allein betrachtet
Y von besonderer Bedeutung in Verbindung mit einer anderen Veröffentlichung derselben Kategorie
A technologischer Hintergrund
O nichtschriftliche Offenbarung
P Zwischenliteratur
T der Erfindung zugrunde liegende Theorien oder Grundsätze

E älteres Patentdokument, das jedoch erst am oder nach dem Anmeldedatum veröffentlicht worden ist
D in der Anmeldung angeführtes Dokument
L aus andern Gründen angeführtes Dokument

& Mitglied der gleichen Patentfamilie, übereinstimmendes Dokument

EPA Form 1503 03 82